# FASCICULE DE BREVET EUROPEEN

(11) **EP 3 806 803 B1**
(45) Date de publication et mention de la délivrance du brevet: **13.12.2023**
(21) Numéro de dépôt: 19732302.5
(22) Date de dépôt: 14.06.2019
(51) Int. Cl.: A61G 5/10, F16M 13/02

(54) **MOYEN DE TRANSPORT POUR PERSONNES À MOBILITÉ RÉDUITE COMPORTANT UN DISPOSITIF DE CONNEXION À UN PIED A SÉRUM, ENSEMBLE DE CONNEXION ASSOCIÉ**
MITTEL ZUM TRANSPORTIEREN VON PERSONEN MIT REDUZIERTER MOBILITÄT MIT EINER VORRICHTUNG ZUR VERBINDUNG MIT EINEM INFUSIONSSTÄNDER, ZUGEHÖRIGE VERBINDUNGSANORDNUNG
MEANS FOR TRANSPORTING PERSONS WITH REDUCED MOBILITY COMPRISING A DEVICE FOR CONNECTING TO AN IV POLE, ASSOCIATED CONNECTION ASSEMBLY

(30) Priorité: 14.06.2018 FR 1855238
(43) Date de publication de la demande: 21.04.2021
(73) Titulaire: Institut Gustave Roussy, 94805 Villejuif Cédex (FR)
(72) Inventeur: DRUBAY, Damien, 92320 Châtillon (FR); NEBBAK, Jean-Marie, 94310 Orly (FR); DAGUET, Joël, 94400 Vitry sur Seine (FR); PECHARD, Serge, 91240 Saint-Michel sur Orge (FR); MAS, Christophe, 94400 Vitry sur Seine (FR)
(74) Mandataire: Cabinet Camus Lebkiri
(86) Numéro de dépôt international: PCT/EP2019/065663
(87) Numéro de publication internationale: WO 2019/238917

(56) Documents cités:
- JP-A- 2016 016 244
- US-A- 4 511 157
- US-A- 4 767 131
- US-A- 5 421 548

## Description

### DOMAINE TECHNIQUE DE L'INVENTION

L'invention concerne de manière générale le domaine des équipements médicaux utilisés notamment pour le transport d'un patient.

L'invention se rapporte plus particulièrement à un moyen de transport pour personnes à mobilité réduite comportant un dispositif de connexion à un pied à sérum. L'invention se rapporte également à un ensemble de connexion comportant ledit moyen de transport connecté à un pied à sérum.

### ÉTAT DE LA TECHNIQUE

Le transport d'un patient nécessitant l'administration en intraveineuse de médicaments peut s'avérer difficile pour le personnel hospitalier. En effet, il est difficile pour une seule personne de pousser simultanément la chaise roulante transportant le patient et le pied à sérum qui supporte les systèmes de perfusion, les pompes ainsi que les pousse-seringues. Afin de pallier ce problème, les différents dispositifs d'administration de médicaments sont transférés, avant chaque trajet, depuis le pied à sérum du patient jusqu'au pied à sérum de la chaise roulante qui est généralement disposé à l'arrière de la chaise roulante.

Cependant, de telles opérations de transfert sont plus ou moins chronophages selon le nombre de dispositifs à transférer. De plus, elles nécessitent une vérification des perfusions après chaque transfert, et leur réglage le cas échéant. En outre, ces opérations sont fréquentes dans la mesure où elles doivent être réalisées au départ d'un service, lors du retour en chambre mais également lors des examens ou des traitements et cela, pour chaque patient. Or, le personnel hospitalier est souvent indisponible si bien qu'une attente dans les unités de soin est souvent observée. En outre, lorsque le nombre de dispositifs d'administration de médicaments transférés est trop important, il existe un risque de basculement de la chaise roulante vers l'arrière. À cela s'ajoutent les risques d'arrachage, de chutes et de bris lors de chaque opération de transfert.

US 4 767 131 A divulgue un moyen de transport pour personnes à mobilité réduite comportant un dispositif de connexion à un pied à sérum.

### RÉSUMÉ DE L'INVENTION

L'invention est exposée dans le jeu de revendications joint. Dans ce contexte, l'invention vise à remédier à tout ou partie des inconvénients de l'état de la technique identifiés ci-dessus, en proposant une solution permettant de réduire le temps et le nombre d'opérations à effectuer avant le transport d'un patient sous perfusions.

Selon un premier aspect, l'invention se rapporte à un moyen de transport pour personnes à mobilité réduite comportant un châssis présentant deux traverses latérales reliant un premier ensemble de roues arrière à un second ensemble de roues avant.

En outre, le moyen de transport comporte un dispositif de connexion à un pied à sérum qui comporte une tige et un support à roulettes fixé à une extrémité de ladite tige.

En particulier, le dispositif de connexion comporte :
∘ un support de fixation fixé au châssis,
∘ un élément de couplage destiné à être solidarisé à la tige du pied à sérum, l'élément de couplage comportant :
   - une pince de serrage présentant un logement adapté pour recevoir la tige et,
   - un élément de serrage adapté pour maintenir la tige dans le logement,
∘ un organe de liaison de l'élément de couplage au support de fixation,
∘ une butée latérale fixée à une des traverses latérales du châssis, la butée latérale étant construite et agencée pour assurer un calage du support à roulettes du pied à sérum par rapport au châssis et pour assurer un positionnement latéral du pied à sérum par rapport au moyen de transport.

Par « pied à sérum », on entend un dispositif destiné à assurer la suspension de dispositifs d'administration de médicaments, appelé également pied à perfusion.

Par « châssis », on entend un assemblage, pouvant être pliable, destiné à supporter notamment les roues, l'assise et le dossier du moyen de transport.

L'invention selon le premier aspect permet de résoudre les problèmes préalablement cités.

Grâce au moyen de transport selon l'invention, il n'est plus nécessaire, avant le transport d'un patient, de transférer tous les dispositifs d'administration de médicaments depuis le pied à sérum du patient vers le pied à sérum du moyen de transport.

En effet, le pied à sérum du patient est directement connecté et solidarisé au châssis du moyen de transport par l'intermédiaire du dispositif de connexion ce qui permet de réduire considérablement le temps, le nombre d'opérations et de manipulations à effectuer avant de transférer un patient. De plus, il n'est pas non plus nécessaire de vérifier les perfusions après chaque transfert, et d'assurer leur réglage le cas échéant ce qui constitue un gain de temps considérable et ce qui permet de réduire l'attente dans les unités de soins. En outre, dans la mesure où il n'est plus nécessaire de transférer les dispositifs d'administration de médicaments d'un pied à sérum à un autre, les risques d'arrachage, de chutes et de bris sont également réduits.

En outre, la butée latérale permet de bloquer les déplacements du pied à sérum par rapport au moyen de transport de manière à stabiliser et à sécuriser le pied à sérum du patient pendant les trajets. En particulier, cela permet d'éviter un basculement du pied à sérum lorsque le moyen de transport est déplacé. En outre, la fixation de la butée latérale au niveau d'au moins une traverse latérale du moyen de transport permet d'assurer un positionnement latéral du pied à sérum par rapport au moyen de transport qui est plus sûr qu'un positionnement à l'arrière ou à l'avant du moyen de transport. Un tel agencement permet d'éviter que la personne chargée de pousser le moyen de transport percute le support à roulettes du pied à sérum au cours du trajet et de lui assurer une bonne visibilité.

Par ailleurs, le dispositif de connexion selon l'invention est simple, rapide à installer et s'adapte à tous types de pied à sérum. Il renforce l'autonomie des patients perfusés, qui peuvent être facilement promenés par un proche ou un tiers. En outre, il nécessite de réaliser peu de modifications sur les moyens de transport existants pour l'installation du dispositif de connexion.

Le moyen de transport selon le premier aspect de l'invention peut également présenter une ou plusieurs des caractéristiques ci-dessous, considérées individuellement ou selon toutes les combinaisons techniquement possibles.

Selon un mode de réalisation non limitatif, la butée latérale présente une forme et des dimensions adaptées pour venir caler deux branches du support à roulettes du pied à sérum.

Selon un mode de réalisation non limitatif, l'organe de liaison comporte au moins un moyen de rappel de l'élément de couplage vers le châssis. Ainsi, lorsque l'organe de liaison n'est pas utilisé, le moyen de rappel repositionne l'organe de liaison dans sa position de repos.

Selon un mode de réalisation non limitatif, l'organe de liaison comporte :
- un axe fixé à la pince de serrage de l'élément de couplage, ledit axe étant monté coulissant à travers ladite au moins une bague du support de fixation,
- une butée radiale fixée audit axe.

Par « radiale », on entend une direction orthogonale à la direction de l'axe de l'organe de liaison.

Selon un mode de réalisation non limitatif, le moyen de rappel est formé par un ressort de compression entourant l'axe de l'organe de liaison, ledit ressort de compression étant configuré pour se comprimer entre une extrémité de ladite au moins une bague et la butée radiale.

Selon un mode de réalisation non limitatif, l'élément de couplage est monté de façon pivotante par rapport à l'organe de liaison.

Selon un mode de réalisation non limitatif, la pince de serrage comporte deux branches présentant chacune une surface interne, le logement étant ménagé dans la surface interne d'une des branches.

Selon un mode de réalisation non limitatif, le logement est construit et agencé pour former au moins deux points de contact entre le logement et la tige.

Selon un mode de réalisation non limitatif, le logement présente une forme de « V ».

Selon un mode de réalisation non limitatif, le moyen de transport comporte au moins un accoudoir destiné à être fixé sur le châssis, ledit au moins un accoudoir étant construit et agencé pour assurer un calage de la tige du pied à sérum par rapport audit châssis.

Selon un mode de réalisation non limitatif, le dispositif de connexion comporte :
- un élément de couplage supplémentaire fixé à l'organe de liaison, l'élément de couplage supplémentaire étant destiné à être solidarisé à la tige d'un pied à sérum supplémentaire et comportant :
   ∘ une pince de serrage présentant un logement adapté pour recevoir une tige du pied à sérum supplémentaire et,
   ∘ un élément de serrage adapté pour maintenir la tige du pied à sérum dans le logement,
- une butée latérale supplémentaire fixée à l'autre traverse latérale du châssis, ladite butée latérale étant construite et agencée pour assurer un calage d'un support à roulettes fixé à une extrémité de la tige du pied à sérum supplémentaire, par rapport au châssis et pour assurer un positionnement latéral du pied à sérum supplémentaire par rapport au moyen de transport.

Selon un mode de réalisation non limitatif, le dispositif de connexion comporte un accoudoir supplémentaire fixé sur le châssis, ledit accoudoir supplémentaire étant construit et agencé pour assurer un calage de la tige du pied à sérum supplémentaire par rapport audit châssis.

Selon un mode de réalisation non limitatif, au moins un des deux accoudoirs est amovible.

Selon un mode de réalisation non limitatif, le moyen de transport se présente sous la forme d'un des moyens de transport choisi dans la liste suivante :
- une chaise roulante,
- un brancard à roulettes,
- un lit à roulettes,
- une poussette.

Selon un deuxième aspect, l'invention se rapporte à un ensemble de connexion d'un pied à sérum à un moyen de transport pour personnes à mobilité réduite selon le premier aspect, ledit ensemble de connexion comportant :
- le moyen de transport,
- le pied à sérum comportant une tige et un support à roulettes fixé à une extrémité de ladite tige,
le moyen de transport étant couplé au pied à sérum au moyen du dispositif de connexion dudit moyen de transport.

### BRÈVE DESCRIPTION DES FIGURES

D'autres caractéristiques et avantages de l'invention ressortiront à la lecture de la description qui suit, en référence aux figures annexées, qui illustrent :
- à la figure 1, une vue de face d'un exemple de moyen de transport pour personnes à mobilité réduite selon un mode de réalisation de l'invention, lorsqu'il est connecté à un pied à sérum,
- à la figure 2, une vue latérale du moyen de transport illustré à la figure 1,
- à la figure 3a, le calage d'une butée latérale du moyen de transport illustré à la figure 1 entre deux branches d'un support à roulettes du pied à sérum,
- à la figure 3b, une vue de face de la butée latérale illustrée à la figure 3a,
- à la figure 3c, une vue de dessus de la butée latérale représentée à la figure 3a,
- à la figure 4a, l'élément de couplage du moyen de transport illustré à la figure 1, lorsqu'il est couplé à la tige du pied à sérum,
- à la figure 4b, une vue de dessus de l'élément de couplage illustré à la figure 4a, en position horizontale, lorsqu'il n'est pas couplé à la tige du pied à sérum,
- à la figure 5a, une vue de dessus du dispositif de connexion du moyen de transport illustré à la figure 1, lorsqu'il est fixé au châssis de la chaise roulante,
- à la figure 5b, une vue de face du dispositif de connexion illustré à la figure 5a, lorsqu'il est au repos,
- à la figure 5c, une vue de dessus du dispositif de connexion illustré à la figure 5a.

### DESCRIPTION D'AU MOINS UN MODE DE REALISATION

L'invention se rapporte à un moyen de transport 20 pour personnes à mobilité réduite comportant un dispositif de connexion 100 adapté pour assurer la connexion entre un pied à sérum 10 et le moyen de transport 20. On rappelle qu'un pied à sérum, également appelé pied à perfusion, est un dispositif utilisé pour supporter des dispositifs d'administration de médicaments tels que des systèmes de perfusion, des pompes ainsi que des pousses seringues nécessaires à l'administration intraveineuse de médicaments à un patient. Avantageusement, le moyen de transport 20 peut se présenter sous la forme d'une chaise roulante, un brancard à roulettes, un lit à roulettes ou encore une poussette pour enfants.

Dans la suite de la description, on admettra que le moyen de transport 20 est une chaise roulante.

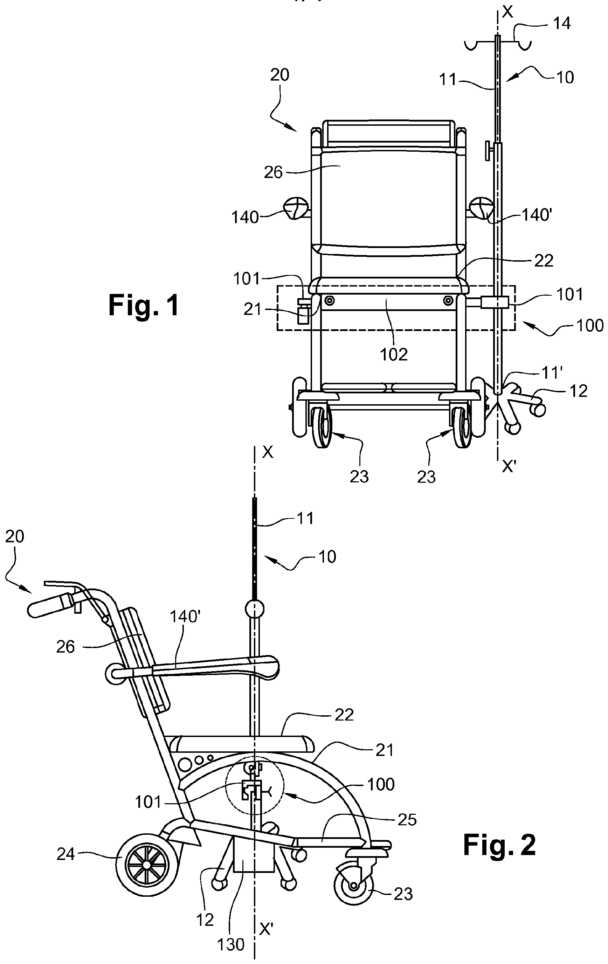

Les figures 1 et 2 représentent respectivement une vue de face et une vue latérale de la chaise roulante 20 selon un mode de réalisation de l'invention lorsqu'elle est connecté au pied à sérum 10.

Le pied à sérum 10 représenté sur les figures 1 et 2 est un exemple de pied à sérum classiquement utilisé dans les hôpitaux.

En référence aux figures 1 et 2, le pied à sérum 10 comporte une tige 11 s'étendant selon un axe longitudinal XX' et un support à roulettes 12 fixé à une extrémité 11' de la tige 11. Au niveau de son autre extrémité, la tige 11 du pied à sérum 10 comporte des crochets 14 sur lesquels sont suspendus les différents dispositifs d'administration de médicaments.

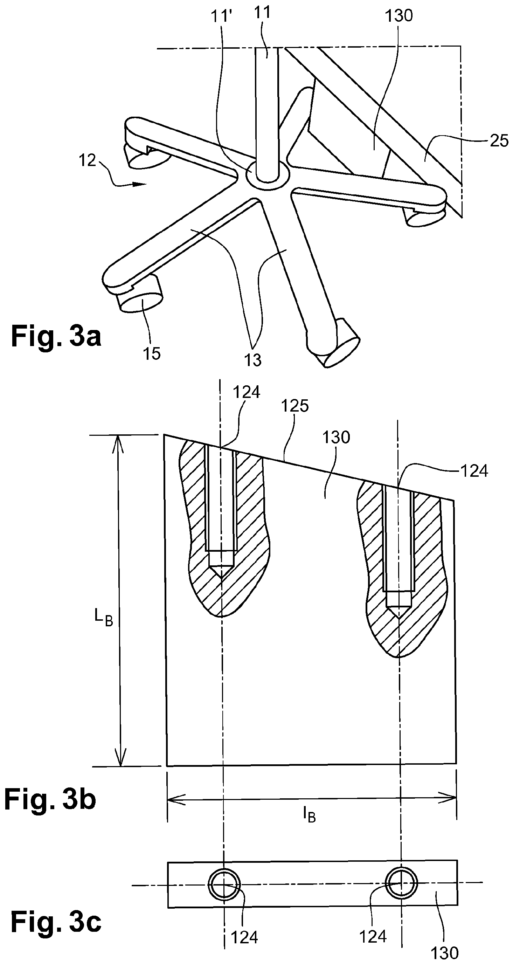

Le support à roulettes 12, représenté de manière plus distinctive à la figure 3a, permet de déplacer facilement le pied à sérum 10 et d'accompagner les déplacements du patient sous perfusion. Le support à roulettes 12 du pied à sérum 10 est formé par une pluralité de branches 13, cinq dans l'exemple illustré à la figure 3a, sur lesquelles sont montées des roulettes 15 destinées à être au contact du sol.

En outre, la chaise roulante 20 comporte un châssis 21 supportant une assise 22, un dossier 26 ainsi qu'un premier ensemble de roues arrière 24 et un second ensemble de roues avant 23. Le premier ensemble de roues arrière 24 et le second ensemble de roues avant 23 sont séparés par des traverses latérales 25 du châssis 21 visibles à la figure 2.

Par ailleurs, le dispositif de connexion 100 comporte :
- un élément de couplage 101,
- un support de fixation 102,
- un organe de liaison 105,
- une butée latérale 130,
- deux accoudoirs 140, 140'.

L'élément de couplage 101 permet de coupler la tige 11 du pied à sérum 10 au châssis 21 de la chaise roulante 20.

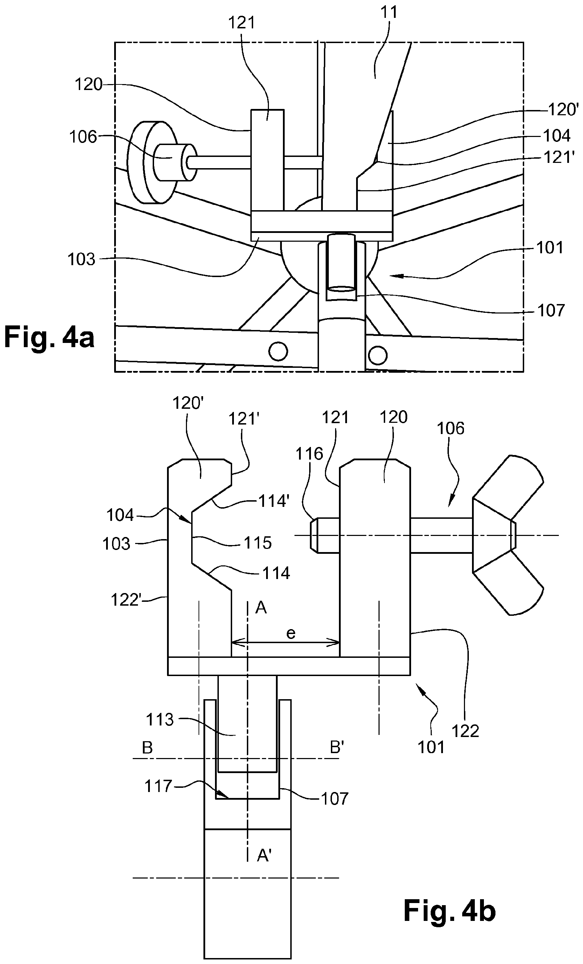

En référence aux figures 4a et 4b, l'élément de couplage 101 comporte une pince de serrage 103 permettant d'enserrer la tige 11. En particulier, la pince de serrage 103 présente deux branches 120, 120' parallèles formant une mâchoire. Selon un mode de réalisation, la pince de serrage 103 est un élément monobloc, par exemple obtenu par moulage. Selon un autre mode de réalisation, la pince de serrage 103 est obtenue par assemblage des deux branches 120, 120' sur une base de liaison. Dans une variante de réalisation, la base de liaison et une des branches forment un élément monobloc, par exemple obtenu par usinage, sur lequel est assemblée l'autre branche.

Par ailleurs, chaque branche 120, 120' de la pince de serrage 103 présente une surface interne 121, 121' et une surface externe 122, 122'. Avantageusement, l'écartement e entre les surfaces internes 121, 121' des branches 120, 120' est adapté pour l'introduction de la tige 11 du pied à sérum 10. Ainsi, l'écartement e est, par exemple, compris dans l'intervalle [10 mm, 60 mm]. Par «écartement» entre les branches, on entend la distance séparant les surfaces internes 121, 121' des branches 120, 120' entre elles. On note que, selon un mode de réalisation de l'invention, l'écartement e peut être modifié par des moyens ad hoc de manière à s'adapter aux dimensions de la tige 11. Avantageusement, les angles saillants des branches 120, 120' de la pince de serrage 103 sont chanfreinés de manière à éviter les risques de blessures si une personne vient à heurter la pince de serrage 103.

En outre, l'épaisseur ainsi que le matériau de la pince de serrage 103 sont choisis de manière à assurer à la pince de serrage 103 une résistance suffisante pour ne pas se déformer et/ou se rompre en cas de choc ou toute autre contrainte à laquelle elle pourrait être soumise. De plus, la pince de serrage 103 est avantageusement réalisée dans un matériau adapté pour ne pas rouiller, en particulier après de nombreuses opérations de nettoyage. Avantageusement, la pince de serrage 103 est réalisée en acier inoxydable.

Par ailleurs, la pince de serrage 103 comporte un logement 104, ménagé au niveau de la surface interne 121' d'une des branches 120, 120' de la pince de serrage 103, adapté pour recevoir la tige 11. Le logement 104 présente une forme et des dimensions adaptées pour former au moins deux points de contact avec la tige 11. Avantageusement, le logement 104 présente globalement une forme de « V », telle que représentée sur la figure 4b. En particulier, le logement 104 présente deux surfaces obliques 114, 114' par rapport à une surface intermédiaire 115 parallèle à la surface externe 122' de la branche 120', la surface intermédiaire 115 séparant les surfaces obliques 114, 114'. Le logement 104 forme alors un point de contact avec la tige 11 avec chaque surface oblique 114 et 114'. Dans une variante de réalisation, le logement 104 ne présente pas de surface intermédiaire 115 ce qui permet de caler des tiges 11 de pieds à sérum 10 de faible diamètre entre les surfaces obliques 114, 114'.

Dans une variante de réalisation non illustrée, la pince de serrage 103 se présente sous la forme d'une pince à grenouillère ou pince à bascule, i.e. une pince munie d'un levier qui permet, selon sa position, de maintenir la pince fermée ou ouverte.

Avantageusement, l'élément de couplage 101 comporte un élément de serrage 106 permettant de maintenir la tige 11 dans le logement 104. Dans le mode de réalisation des figures 4a et 4b, l'élément de serrage 106 est formé par une vis de serrage adaptée pour être vissée dans un orifice (non visible) taraudé traversant la branche 120 opposée à la branche 120' dans laquelle est ménagé le logement 104. Le maintien de la tige 11 dans le logement 104 est assuré par vissage de l'élément de serrage 106 dans l'orifice jusqu'à ce que l'extrémité libre 116 de l'élément de serrage 106 vienne en appui contre la tige 11 et exerce ainsi un effort suffisant pour éviter un désengagement de la tige 11 du logement 104. Dès lors, trois points de contact entre la tige 11 et l'élément de couplage 101 sont assurés par les deux surfaces obliques 114 et 114' et l'extrémité libre 116 de l'élément de serrage 106. Dans une variante de réalisation non illustrée, l'extrémité libre 116 de l'élément de serrage 106 forme un V de manière à ce que la tige 11 ait un point de contact supplémentaire avec l'élément de serrage 106 ce qui permet d'améliorer son maintien dans le logement 104.

Avantageusement, l'extrémité libre 116 de l'élément de serrage 106, ou la totalité de l'élément de serrage 106, présente un revêtement compressible, par exemple en caoutchouc ou en plastique. Ainsi, lors du vissage de l'élément de serrage 106, le revêtement se comprime contre la tige 11 ce qui permet d'améliorer le maintien de la tige 11 dans le logement 104.

Par ailleurs, le support de fixation 102 assure la fixation du dispositif de connexion 100 sur le châssis 21 de la chaise roulante 20.

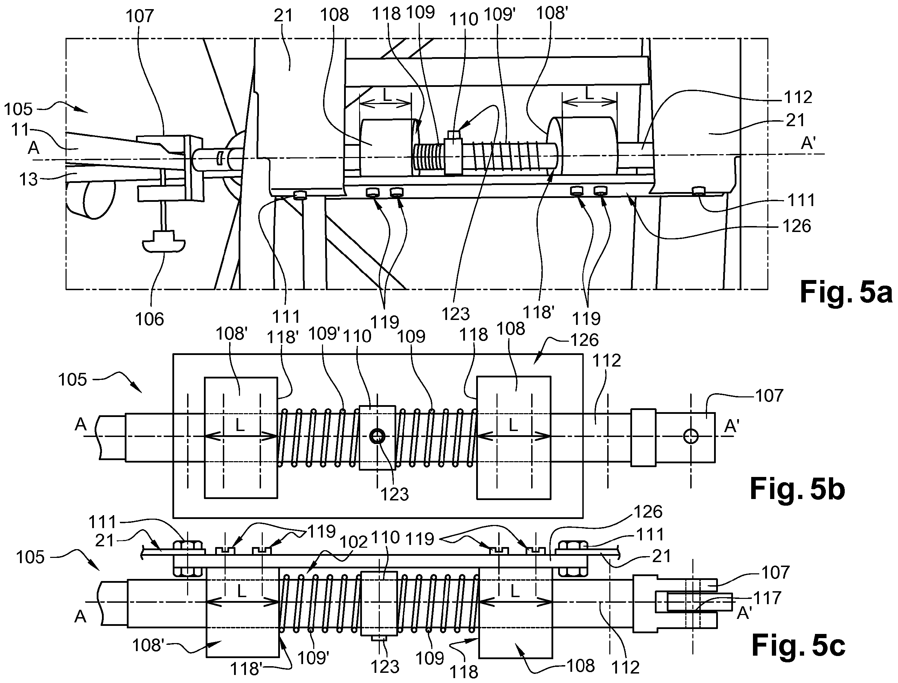

Selon le mode de réalisation illustré aux figures 5a à 5c, le support de fixation 102 comporte une plaque 126 rectangulaire fixée sous l'assise 22 de la chaise roulante 20 au moyen de vis de fixation 111 traversant le châssis 21 et ladite plaque 126. Dans une variante de réalisation non représentée, le support de fixation 102 est fixé sur le châssis 21 par soudure.

Avantageusement, l'épaisseur et le matériau de la plaque 126 sont choisis de manière à lui assurer une résistance suffisante pour ne pas se déformer et/ou se rompre lors de son utilisation. Ainsi, la plaque 126 est par exemple réalisée en acier inoxydable ou en aluminium. En outre, la plaque 126 présente par exemple une épaisseur comprise dans l'intervalle [5 mm, 40 mm]. On note que la forme et les dimensions de la plaque 126 peuvent varier selon la forme du châssis 21 et/ou la largeur de la chaise roulante 20. En effet, une chaise roulante destinée à transporter un enfant présente une largeur environ égale à 300 mm tandis qu'une chaise roulante destinée à transporter une personne obèse présente une largeur d'environ 800mm.

Par ailleurs, le support de fixation 102 comporte au moins une bague fixée à la plaque 126. Dans le mode de réalisation illustré aux figures 5a, 5b et 5c, le support de fixation 102 comporte deux bagues 108 et 108'. Dans un autre mode de réalisation non représenté, le support de fixation 102 comporte plus de deux bagues. Les bagues 108 et 108' sont fixées à la plaque 126 par des vis de fixation 119, ici deux vis de fixation 119 par bague. Bien entendu, les bagues 108, 108' peuvent être fixées à la plaque 126 par un autre moyen de fixation.

De plus, les bagues 108, 108' présentent un diamètre interne adapté pour recevoir un axe 112 de l'organe de liaison 105 et pour assurer son coulissement à travers lesdites bagues 108, 108'. Avantageusement, les bagues 108, 108' sont réalisées dans un matériau présentant un faible coefficient de friction de manière à faciliter le coulissement de l'axe 112. Avantageusement, les bagues 108, 108' sont réalisées en polytétrafluoroéthylène, connu sous le nom de téflon^{®}. En outre, les bagues 108, 108' présentent une longueur L et une épaisseur adaptées pour leur assurer une résistance suffisante aux contraintes auxquelles elles sont soumises. Par « épaisseur » des bagues, on entend la distance séparant leur diamètre externe de leur diamètre interne. Selon un mode de réalisation, les bagues 108, 108' présentent une longueur L comprise dans l'intervalle [49 mm, 51 mm]. En outre, les bagues 108, 108' présentent par exemple une épaisseur comprise dans l'intervalle [64 mm, 66 mm].

Par ailleurs, l'organe de liaison 105 assure le couplage de l'élément de couplage 101 au support de fixation 102. En référence aux figures 5a à 5c, l'organe de liaison 105 comporte un axe 112 fixé à la pince de serrage 103 de l'élément de couplage 101. Selon le mode de réalisation des figures 5a, 5b et 5c, l'axe 112 se présente sous la forme d'un élément tubulaire s'étendant selon un axe AA'.

Dans le mode de réalisation de la figure 4b, l'axe 112 est plein. Dans ce mode de réalisation, l'axe 112 comporte une extrémité 107 en forme de U de manière à former une lumière 117 apte à recevoir une extrémité 113 de la pince de serrage 103. L'extrémité 107 de l'axe 112 peut être obtenue par usinage, directement dans l'axe 112. Dans une variante de réalisation, l'extrémité 107 de l'axe 112 peut se présenter sous la forme d'un embout qui est fixé au bout de l'axe 112 par de moyens de fixation ce qui permet d'éviter de remplacer la totalité de l'axe 112 en cas de cassure au niveau de ladite extrémité 107.

Avantageusement, la pince de serrage 103 est solidarisée à l'extrémité 107 de l'axe 112 au moyen d'un élément traversant, par exemple une goupille, qui traverse à la fois l'extrémité 107 de l'axe 112 et l'extrémité 113 de la pince de serrage 103. Grâce à la goupille, la pince de serrage 103 est montée pivotante par rapport à l'axe 112. Plus précisément, la pince de serrage 103 est adaptée pour pivoter autour d'un axe BB' perpendiculaire à l'axe AA' selon lequel s'étend l'axe 112. Ainsi, lorsque la pince de serrage 103 est désolidarisée de la tige 11 du pied de sérum 10, la pince de serrage 103 pivote naturellement vers une position verticale orientée vers le sol sous l'effet de son poids. Un tel système permet d'améliorer la sécurité du dispositif de connexion 100 en limitant les risques de blessures susceptibles de survenir si une personne venait à heurter la pince de serrage 103 lorsque celle-ci n'est pas reliée à la tige 11 du pied à sérum 10. À noter que la pince de serrage 103 est positionnée à l'horizontale lorsqu'elle est couplée à la tige 11.

Dans une variante de réalisation non représentée, l'axe 112 est creux. Dans ce cas, l'extrémité 107 en forme de U se présente sous la forme d'un embout fixé au bout de l'axe 112 par une goupille ou par une vis traversant l'axe 112 et l'embout. Dans cette variante de réalisation, la pince de serrage 103 pivote également par rapport à l'axe 112.

Par ailleurs, les dimensions ainsi que le matériau de l'axe 112 sont choisis de manière à lui assurer une résistance mécanique suffisante pour supporter les contraintes auxquelles il est soumis. Avantageusement, l'axe 112 est en acier inoxydable lorsqu'il est creux et est en aluminium lorsqu'il est plein. En outre, l'axe 112 s'étend sur une longueur adaptée aux dimensions de la chaise roulante 20, par exemple comprise dans l'intervalle [450 mm, 460 mm]. Naturellement, la forme et les dimensions de l'axe 112 peuvent varier selon la forme du châssis 21 et/ou la largeur de la chaise roulante 20.

L'organe de liaison 105 comporte une butée radiale 110 fixée à l'axe 112. Selon le mode de réalisation des figures 5a, 5b et 5c, la butée radiale 110 est formée par un anneau entourant l'axe 112 disposé au niveau d'une portion centrale de l'axe 112. Ainsi, l'anneau présente un diamètre interne supérieur au diamètre externe de l'axe 112. Avantageusement, la butée radiale 110 est fixée à l'axe 112 par une vis de fixation 123. La butée radiale 110 et l'axe 112 peuvent former un élément monobloc, par exemple par usinage, ou être assemblés, par exemple par soudure.

Par ailleurs, l'organe de liaison 105 comporte au moins un moyen de rappel 109 adapté pour exercer une force de rappel sur l'élément de couplage 101 qui l'oriente en direction du châssis 21 de la chaise roulante 20. Pour ce faire, le moyen de rappel 109 exerce une force de rappel sur l'axe 112 qui est fixé à la pince de serrage 103. Selon le mode de réalisation des figures 5a, 5b et 5c, le moyen de rappel 109 est formé par un ressort de compression entourant l'axe 112. Le ressort de compression est adapté pour se comprimer entre une extrémité 118 d'une bague 108 et la butée radiale 110 de l'organe de liaison 105. La compression du ressort de compression permet de rétracter l'axe 112 et ainsi l'élément de couplage 101 sous l'assise 22 lorsque la tige 11 est désolidarisée de l'élément de couplage 101. En d'autres termes, le ressort de compression tire en permanence sur la pince de serrage 103 pour l'orienter sous l'assise 22 de la chaise roulante 20. Cela permet d'éviter qu'une personne passant à proximité de la chaise roulante 20 ne se blesse en heurtant la pince de serrage 103. Avantageusement, le ressort de compression est souple afin que la pince de serrage 103 ne se rétracte pas brutalement sous l'assise 22 lorsqu'un opérateur libère la tige 11 de la pince de serrage 103, par exemple en Inox. En outre, le ressort de compression présente par exemple une longueur au repos comprise dans l'intervalle [50 mm, 60 mm].

Selon un mode de réalisation non représenté, le ressort de compression est remplacé par un ensemble de vérins qui assurent la même fonction. Avantageusement, les moyens de rappel 109 et 109' ainsi que la butée radiale 110 sont recouverts par un soufflet ou encore par un cache, par exemple en PVC afin de les protéger.

Selon un autre mode de réalisation, la rétractation de la pince de serrage 103 sous la chaise roulante 20 est réalisée manuellement par le personnel ou le tiers accompagnant le patient.

Dans un mode de réalisation non illustré, la tige 11 du pied à sérum 10 est couplée à la chaise roulante 20 au moyen de deux dispositifs de connexion 100. Dans ce cas, le support de fixation 102 d'un autre dispositif de connexion 100 est par exemple fixé sur une des traverses latérales 25 de la chaise roulante 20.

Par ailleurs, la butée latérale 130 du dispositif de connexion 100 permet d'assurer le calage du support à roulettes 12 par rapport au châssis 21 de la chaise roulante 20 ainsi qu'un positionnement latéral du pied à sérum 10 par rapport à la chaise roulante 20. A cet effet, la butée latérale 130 est fixée à au moins une des traverses latérales 25 de la chaise roulante 20, entre une roue avant 23 et une roue arrière 24 de la chaise roulante 20.

En référence aux figures 3a, 3b et 3c, la butée latérale 130 présente globalement une forme rectangulaire. En particulier, la butée latérale 130 présente une face supérieure oblique 125 adaptée pour suivre l'inclinaison de la traverse latérale 25 de la chaise roulante 20 sur laquelle la butée latérale 130 est montée.

Par ailleurs, la butée latérale 130 présente une longueur L_{B} et une largeur l_{B} adaptées pour un calage de la butée latérale 130 entre deux branches 13 du support à roulettes 12. La longueur L_{B} de la butée latérale 130 peut varier selon l'inclinaison de la traverse latérale 25. La butée latérale 130 présente par exemple une longueur L_{B} comprise dans l'intervalle [30 mm, 300 mm] et une largeur l_{B} comprise dans l'intervalle [100 mm, 180 mm].

La fixation de la butée latérale 130 sur la traverse latérale 25 est réalisée au moyen de vis de fixation 124, ici deux vis de fixation, traversant ladite traverse latérale 25 et la butée latérale 130 dans le sens de la longueur L_{B} de la butée latérale 130. De manière avantageuse, l'épaisseur de la butée latérale 130 est choisie de sorte à pouvoir y introduire les vis de fixation 124.

Avantageusement, la butée latérale 130 est réalisée dans un matériau permettant d'absorber le bruit susceptible d'être généré par le contact des branches 13 du support à roulettes 12 avec la butée latérale 130 et pour ne pas rouiller, en particulier après plusieurs opérations de nettoyage. La butée latérale 130 est par exemple en polytétrafluoroéthylène, connu sous le nom de téflon^{®}.

Dans une variante de réalisation non illustrée, la butée latérale 130 est réalisée dans un matériau métallique, tel que l'acier ou l'aluminium. Avantageusement, la butée latérale 130 peut alors être soudée sur la traverse latérale 25 de la chaise roulante 20.

En outre, l'épaisseur ainsi que le matériau de la butée latérale 130 sont choisis de manière que la butée latérale 130 présente une résistance mécanique suffisante pour supporter les contraintes auxquelles elle est soumise. À titre d'exemple, l'épaisseur de la butée latérale 130 est comprise dans l'intervalle [10 mm, 40 mm].

De manière avantageuse, les accoudoirs 140, 140' du dispositif de connexion 100 forment un appui pour le coude ou le bras d'un patient tout en assurant le calage de la tige 11 du pied à sérum 10. Les accoudoirs 140, 140' sont fixés au châssis 21 de la chaise roulante 20 par exemple au moyen de vis de fixation traversant le châssis 21. Avantageusement, les accoudoirs 140, 140' sont amovibles. Selon le mode de réalisation illustré à la figure 1, le dispositif de connexion 100 comporte deux accoudoirs 140, 140'. Dans une variante de réalisation, le dispositif de connexion 100 ne comporte qu'un seul accoudoir.

Par ailleurs, selon un second mode de réalisation, le dispositif de connexion 100 comporte :
- deux éléments de couplage 101,
- un support de fixation 102,
- un organe de liaison 105,
- deux butées latérales 130,
- deux accoudoirs 140, 140'.

Un tel mode de réalisation est particulièrement avantageux puisqu'il permet, à partir d'un seul dispositif de connexion 100, de connecter deux pieds à sérum 10 à la même chaise roulante 20.

Dans ce mode de réalisation, une butée latérale 130 est fixée sur chaque traverse latérale 25 de la chaise roulante 20 de manière à assurer le calage du support à roulettes 12 de chaque pied à sérum 10 par rapport au châssis 21 de la chaise roulante 20. En d'autres termes, en plus de la butée latérale 130 décrite précédemment, une butée latérale 130 supplémentaire est fixée sur l'autre traverse latérale 25 afin d'assurer le calage d'un pied à sérum 10 supplémentaire.

En outre, comme on peut le voir sur la figure 1, un élément de couplage 101 est fixé au niveau de chaque extrémité 107 de l'axe 112 de l'organe de liaison 105. Ainsi, en plus de l'élément de couplage 101 décrit précédemment, un élément de couplage 130 supplémentaire est fixé sur l'autre extrémité 107 de l'axe 112 de l'organe de liaison 105. En d'autres termes, deux pinces de serrage 103 ainsi que deux éléments de serrage 106 sont agencées de part et d'autre de l'axe 112.

Par ailleurs, dans ce mode de réalisation, l'organe de liaison 105 comporte deux moyens de rappel 109 et 109' tels qu'illustrés aux figures 5a, 5b et 5c. Les moyens de rappels 109 et 109' sont formés par des ressorts de compression disposés de part et d'autre de la butée radiale 110. Chaque ressort de compression est adapté pour se comprimer entre une des bagues 108, 108' et la butée radiale 110 de manière à exercer une force de rappel sur chaque pince de serrage 103. La tige 11 de chaque pied à sérum 10 est calée contre un des accoudoirs 140, 140' de la chaise roulante 20.

Les modes de réalisation décrits ci-dessus ne sont nullement limitatifs de sorte que des modifications peuvent y être apportées sans sortir du cadre de l'invention.

## Revendications

1. Moyen de transport (20) pour personnes à mobilité réduite comportant un châssis (21) présentant deux traverses latérales (25) reliant un premier ensemble de roues arrières (24) à un second ensemble de roues avant (23), ledit moyen de transport (20) étant **caractérisé en ce qu'**il comporte un dispositif de connexion (100) à un pied à sérum (10) pour un positionnement latéral dudit pied à sérum, ledit pied à sérum (10) comportant une tige (11) et un support à roulettes (12) fixé à une extrémité (11') de ladite tige (11), le dispositif de connexion (100) comportant :
∘ un support de fixation (102) fixé au châssis (21),
∘ un élément de couplage (101) destiné à être solidarisé à la tige (11), l'élément de couplage (101) comportant :
- une pince de serrage (103) présentant un logement (104) adapté pour recevoir la tige (11) et,
- un élément de serrage (106) adapté pour maintenir la tige (11) dans le logement (104),
∘ un organe de liaison (105) de l'élément de couplage (101) au support de fixation (102),
∘ une butée latérale (130) fixée à une des traverses latérales (25) du châssis (21), ladite butée latérale (130) étant construite et agencée pour assurer un calage du support à roulettes (12) par rapport au châssis (21).

2. Moyen de transport (20) selon la revendication précédente, **caractérisé en ce que** la pince de serrage (103) comporte deux branches (120, 120') présentant chacune une surface interne (121, 121'), le logement (104) étant ménagé dans une surface interne (121') d'une des branches (120, 120').

3. Moyen de transport (20) selon l'une des revendications précédentes, **caractérisé en ce que** le logement (104) est construit et agencé pour former au moins deux points de contact entre le logement (104) et la tige (11).

4. Moyen de transport (20) selon l'une des revendications précédentes, **caractérisé en ce que** le logement (104) présente une forme de « V ».

5. Moyen de transport (20) selon l'une des revendications précédentes, **caractérisé en ce que** le support de fixation (102) comporte :
- une plaque (126) destinée à être fixée directement sur le châssis (21) de la chaise roulante (20),
- au moins une bague (108, 108') fixée à ladite plaque (126).

6. Moyen de transport (20) selon l'une des revendications précédentes, **caractérisé en ce que** l'organe de liaison (105) comporte au moins un moyen de rappel (109, 109') de l'élément de couplage (101) vers le châssis (21) de la chaise roulante (20).

7. Moyen de transport (20) selon les revendications 5 à 6, **caractérisé en ce que** l'organe de liaison (105) comporte :
∘ un axe (112) fixé à la pince de serrage (103) de l'élément de couplage (101), ledit axe (112) étant monté coulissant à travers ladite au moins une bague (108, 108') du support de fixation (102),
∘ une butée radiale (110) fixée audit axe (112).

8. Moyen de transport (20) selon la revendication précédente, **caractérisé en ce que** le moyen de rappel (109, 109') est formé par un ressort de compression (109, 109') entourant l'axe (112), ledit ressort de compression (109, 109') étant adapté pour se comprimer entre une extrémité (118, 118') de ladite au moins une bague (108, 108') et la butée radiale (110).

9. Moyen de transport (20) selon l'une des revendications précédentes, **caractérisé en ce que** la butée latérale (130) présente une forme et des dimensions adaptées pour venir caler deux branches (13) du support à roulettes (12) du pied à sérum (10).

10. Moyen de transport (20) selon l'une des revendications précédentes, **caractérisé en ce que** le dispositif de connexion (100) comporte au moins un accoudoir (140, 140') fixé sur le châssis (21), ledit au moins un accoudoir (140, 140') étant construit et agencé pour assurer un calage de la tige (11) du pied à sérum (10) par rapport audit châssis (21).

11. Moyen de transport (20) selon l'une des revendications précédentes, **caractérisé en ce que** le dispositif de connexion (100) comporte :
- un élément de couplage (101) supplémentaire fixé à l'organe de liaison (105), ledit élément de couplage (101) supplémentaire étant destiné à être solidarisé à la tige (11) d'un pied à sérum (10) supplémentaire et comportant :
∘ une pince de serrage (103) présentant un logement (104) adapté pour recevoir une tige (11) du pied à sérum (10) supplémentaire et,
∘ un élément de serrage (106) adapté pour maintenir la tige (11) du pied à sérum (10) dans le logement (104),
- une butée latérale (130) supplémentaire fixée à l'autre traverse latérale (25) du châssis (21), ladite butée latérale (130) étant construite et agencée pour assurer un calage d'un support à roulettes (12) du pied à sérum (10) supplémentaire, par rapport au châssis (21) et pour assurer un positionnement latéral du pied à sérum (10) supplémentaire par rapport au moyen de transport (20).

12. Moyen de transport (20) selon la revendication précédente, **caractérisé en ce qu'**il comporte un accoudoir supplémentaire (140') fixé sur le châssis (21), ledit accoudoir supplémentaire (140') étant construit et agencé pour assurer un calage de la tige (11) du pied à sérum (10) supplémentaire par rapport audit châssis (21).

13. Moyen de transport (20) selon l'une quelconque des revendications précédentes, **caractérisé en ce qu'**il se présente sous la forme d'un des moyens de transport choisi parmi la liste suivante :
- une chaise roulante,
- un brancard à roulettes,
- un lit à roulettes,
- une poussette.

14. Ensemble de connexion d'un pied à sérum (10) au moyen de transport (20) pour personnes à mobilité réduite selon l'une quelconque des revendications précédentes, ledit ensemble de connexion comportant :
- le moyen de transport (20),
- le pied à sérum (10) comportant une tige (11) et un support à roulettes (12) fixé à une extrémité (11') de ladite tige (11),
le moyen de transport (20) étant couplé au pied à sérum (10) au moyen du dispositif de connexion (100) dudit moyen de transport (20).

## Patentansprüche

1. Transportmittel (20) für Personen mit eingeschränkter Mobilität, das ein Fahrgestell (21) mit zwei seitlichen Querträgern (25) umfasst, die eine erste Gruppe von Hinterrädern (24) mit einer zweiten Gruppe von Vorderrädern (23) verbinden, wobei das Transportmittel (20) **dadurch gekennzeichnet ist, dass** es eine Verbindungsvorrichtung (100) zu einem Infusionsständer (10) für eine seitliche Positionierung des Infusionsständers umfasst, wobei der Infusionsständer (10) eine Stange (11) und einen Rollenträger (12) umfasst, der an einem Ende (11') der Stange (11) befestigt ist, wobei die Verbindungsvorrichtung (100) umfasst:
∘ eine Befestigungshalterung (102), die am Fahrgestell (21) befestigt ist,
∘ ein Kopplungselement (101), das dazu bestimmt ist, fest mit der Stange (11) verbunden zu werden,
wobei das Kopplungselement (101) umfasst:
- eine Spannzange (103) mit einer Aufnahme (104), das zur Aufnahme der Stange (11) geeignet ist, und
- ein Klemmelement (106), das so beschaffen ist, dass es die Stange (11) in der Aufnahme (104) hält,
∘ ein Verbindungsglied (105) des Kopplungselements (101) mit der Befestigungshalterung (102),
∘ einen Seitenanschlag (130), der an einem der Seitenquerträger (25) des Fahrgestells (21) fixiert ist, wobei der Seitenanschlag (130) so ausgebildet und angeordnet ist, dass er eine Verkeilung des Rollenträgers (12) in Bezug zum Fahrgestell (21) bewirkt.

2. Transportmittel (20) nach vorhergehendem Anspruch, **dadurch gekennzeichnet, dass** die Spannzange (103) zwei Schenkel (120, 120') umfasst, die jeweils eine Innenfläche (121, 121') aufweisen, wobei die Aufnahme (104) in einer Innenfläche (121') eines der Schenkel (120, 120') ausgebildet ist.

3. Transportmittel (20) nach einem der vorhergehenden Ansprüche, **dadurch gekennzeichnet, dass** die Aufnahme (104) so ausgebildet und angeordnet ist, dass sie mindestens zwei Kontaktpunkte zwischen der Aufnahme (104) und der Stange (11) bildet.

4. Transportmittel (20) nach einem der vorhergehenden Ansprüche, **dadurch gekennzeichnet, dass** die Aufnahme (104) eine "V"-Form aufweist.

5. Transportmittel (20) nach einem der vorhergehenden Ansprüche, **dadurch gekennzeichnet, dass** die Befestigungshalterung (102) umfasst:
- eine Platte (126), die dazu bestimmt ist, direkt am Rahmen (21) des Rollstuhls (20) befestigt zu werden,
- mindestens einen Ring (108, 108'), der an der Platte (126) befestigt ist.

6. Transportmittel (20) nach einem der vorhergehenden Ansprüche, **dadurch gekennzeichnet, dass** das Verbindungsglied (105) mindestens ein Rückstellmittel (109, 109') des Kopplungselements (101) in Richtung des Rahmens (21) des Rollstuhls (20) umfasst.

7. Transportmittel (20) nach den Ansprüchen 5 bis 6, **dadurch gekennzeichnet, dass** das Verbindungsglied (105) umfasst:
∘ einen Stift (112), der an der Spannzange (103) des Kopplungselements (101) befestigt ist, wobei der Stift (112) gleitend durch den mindestens einen Ring (108, 108') der Befestigungshalterung (102) montiert ist,
∘ einen radialen Anschlag (110), der an dem Stift (112) befestigt ist.

8. Transportmittel (20) nach vorhergehendem Anspruch, **dadurch gekennzeichnet, dass** das Rückstellmittel (109, 109') durch eine den Stift (112) umgebende Druckfeder (109, 109') gebildet wird, wobei die Druckfeder (109, 109') dazu geeignet ist, sich zwischen einem Ende (118, 118') des mindestens einen Rings (108, 108') und dem radialen Anschlag (110) zusammenzudrücken.

9. Transportmittel (20) nach einem der vorhergehenden Ansprüche, **dadurch gekennzeichnet, dass** der Seitenanschlag (130) eine Form und Abmessungen aufweist, die geeignet sind, zwei Schenkel (13) des Rollenträgers (12) des Infusionsständers (10) zu verkeilen.

10. Transportmittel (20) nach einem der vorhergehenden Ansprüche, **dadurch gekennzeichnet, dass** die Verbindungsvorrichtung (100) mindestens eine Armlehne (140, 140`) umfasst, die an dem Rahmen (21) befestigt ist, wobei die mindestens eine Armlehne (140, 140') so erstellt und angeordnet ist, dass sie eine Verkeilung der Stange (11) des Infusionsständers (10) in Bezug zum Rahmen (21) gewährleistet.

11. Transportmittel (20) nach einem der vorhergehenden Ansprüche, **dadurch gekennzeichnet, dass** die Verbindungsvorrichtung (100) umfasst:
- ein zusätzliches Kopplungselement (101), das an dem Verbindungsglied (105) befestigt ist, wobei das zusätzliche Kopplungselement (101) dazu bestimmt ist, fest mit der Stange (11) eines zusätzlichen Infustionsständers (10) verbunden zu werden und umfasst:
∘ eine Spannzange (103) mit einer Aufnahme (104), die geeignet ist, eine Stange (11) des zusätzlichen Infusionsständers (10) aufzunehmen, und
∘ ein Klemmelement (106), das dazu geeignet ist, die Stange (11) des Infusionsständers (10) in der Aufnahme (104) zu halten,
- einen zusätzlichen Seitenanschlag (130), der an dem anderen Seitenquerträger (25) des Rahmens (21) befestigt ist, wobei der Seitenanschlag (130) so erstellt und angeordnet ist, dass er einen Rollenträger (12) des zusätzlichen Infusionsständers (10) in Bezug auf den Rahmen (21) verkeilt und eine seitliche Positionierung des zusätzlichen Infusionsständers (10) in Bezug auf das Transportmittel (20) sicherstellt.

12. Transportmittel (20) nach vorhergehendem Anspruch, **dadurch gekennzeichnet, dass** es eine zusätzliche Armlehne (140`) aufweist, die am Rahmen (21) befestigt ist, wobei die zusätzliche Armlehne (140') so erstellt und angeordnet ist, dass sie eine Verkeilung der Stange (11) des zusätzlichen Infustionsständers (10) in Bezug auf den Rahmen (21) gewährleistet.

13. Transportmittel (20) nach einem der vorhergehenden Ansprüche, **dadurch gekennzeichnet, dass** es in Form eines der Transportmittel vorliegt, die aus folgender Liste ausgewählt werden:
- ein Rollstuhl,
- eine Rolltrage,
- ein Bett auf Rollen,
- ein Kinderwagen.

14. Verbindungssystem eines Infusionsständers (10) und eines Transportmittels (20) für Personen mit eingeschränkter Mobilität nach einem der vorhergehenden Ansprüche, wobei das Verbindungssytem umfasst:
- das Transportmittel (20),
- den Infusionsständer (10) mit einer Stange (11) und einem Rollenträger (12), der an einem Ende (11') der Stange (11) befestigt ist,
wobei das Transportmittel (20) mittels dem Verbindungssystem (100) des Transportmittels (20) mit dem Infusionsständer (10) gekoppelt ist.

## Claims

1. A transporting means (20) for persons with reduced mobility comprising a chassis (21) having two side cross members (25) connecting a first assembly of rear wheels (24) to a second assembly of front wheels (23), said transporting means (20) being **characterised in that** it comprises a connection device (100) for connecting to a serum holder (10) for a lateral positioning of the serum holder (10), said serum holder (10) comprising a rod (11) and a wheeled support (12) attached to one end (11') of said rod (11), the connection device (100) comprising:
o an attachment support (102) attached to the chassis (21),
o a coupling element (101) for being secured to the rod (11), the coupling element (101) comprising:
- a clamp (103) having a housing (104) adapted to receive the rod (11) and,
- a clamping element (106) adapted to hold the rod (11) in the housing (104),
o a connection member (105) for connecting the coupling element (101) to the attachment support (102),
o a side stop (130) attached to one of the side cross members (25) of the chassis (21), said side stop (130) being constructed and arranged to ensure locking of the wheeled support (12) with respect to the chassis (21).

2. The transporting means (20) according to the preceding claim, **characterised in that** the clamp (103) comprises two branches (120, 120') each having an inner surface (121, 121'), the housing (104) being provided in an inner surface (121') of one of the branches (120, 120').

3. The transporting means (20) according to one of the preceding claims, **characterised in that** the housing (104) is constructed and arranged to form at least two contact points between the housing (104) and the rod (11).

4. The transporting means (20) according to one of the preceding claims, **characterised in that** the housing (104) has a "V" shape.

5. The transporting means (20) according to one of the preceding claims, **characterised in that** the attachment support (102) comprises:
- a plate (126) for being directly attached to the chassis (21) of the wheelchair (20),
- at least one ring (108, 108') attached to said plate (126).

6. The transporting means (20) according to one of the preceding claims, **characterised in that** the connection member (105) comprises at least one return means (109, 109') for returning the coupling element (101) to the chassis (21) of the wheelchair (20).

7. The transporting means (20) according to claims 5 to 6, **characterised in that** the connection member (105) includes:
o an axis (112) attached to the clamp (103) of the coupling element (101), said axis (112) being slidably mounted through said at least one ring (108, 108') of the attachment support (102),
o a radial stop (110) attached to said axis (112).

8. The transporting means (20) according to the preceding claim, **characterised in that** the return means (109, 109') is formed by a compression spring (109, 109') surrounding the axis (112), said compression spring (109, 109') being adapted to be compressed between one end (118, 118') of said at least one ring (108, 108') and the radial stop (110).

9. The transporting means (20) according to one of the preceding claims, **characterised in that** the side stop (130) has a shape and dimensions adapted to lock two branches (13) of the wheeled support (12) of the serum holder (10).

10. The transporting means (20) according to one of the preceding claims, **characterised in that** the connection device (100) comprises at least one armrest (140, 140') attached to the chassis (21), said at least one armrest (140, 140') being constructed and arranged to ensure locking of the rod (11) of the serum holder (10) relative to said chassis (21).

11. The transporting means (20) according to one of the preceding claims, **characterised in that** the connection device (100) comprises:
- an additional coupling element (101) attached to the connection member (105), said additional coupling element (101) being for being secured to the rod (11) of an additional serum holder (10) and including:
o a clamp (103) having a housing (104) adapted to receive a rod (11) of the additional serum holder (10) and,
o a clamping element (106) adapted to hold the rod (11) of the serum holder (10) in the housing (104),
- an additional side stop (130) attached to the other side cross member (25) of the chassis (21), said side stop (130) being constructed and arranged to ensure locking of a wheeled support (12) of the additional serum holder (10) relative to the chassis (21) and to ensure lateral positioning of the additional serum holder (10) relative to the transport means (20).

12. The transporting means (20) according to the preceding claim, **characterised in that** it includes an additional armrest (140') attached to the chassis (21), said additional armrest (140') being constructed and arranged to ensure locking of the rod (11) of the additional serum holder (10) relative to said chassis (21).

13. The transporting means (20) according to any one of the preceding claims, **characterised in that** it is in the form of one of the transporting means chosen from the following list:
- a wheelchair,
- a wheeled stretcher,
- a wheeled bed,
- a pram.

14. An assembly for connecting a serum holder (10) to the transporting means (20) for persons with reduced mobility according to any of the preceding claims, said connection assembly comprising:
- the transporting means (20),
- the serum holder (10) including a rod (11) and a wheeled support (12) attached to one end (11') of said rod (11),
the transporting means (20) being coupled to the serum holder (10) by means of the connection device (100) of said transporting means (20).
